# EUROPÄISCHE PATENTSCHRIFT

(11) **EP 0 850 931 B1**
(45) Veröffentlichungstag und Bekanntmachung des Hinweises auf die Patenterteilung: **02.05.2003**
(21) Anmeldenummer: 97122627.9
(22) Anmeldetag: 22.12.1997
(51) Int. Cl.: C07D 217/20

(54) **Verfahren zur Herstellung optisch aktiver (R oder S)-1-(4-Methoxy-benzyl)-1,2,3,4,5,6,7,8-Octahydro-isochinoline**
Process for the preparation of optically active (R or S)-1-(4-methoxy-benzyl)-1,2,3,4,5,6,7,8-octahydro-isoquinolines
Procédé pour la préparation de (R ou S)-1-(4-méthoxy-benzyl)-1,2,3,4,5,6,7,8-octahydro-isoquinolines optiquement actives

(30) Priorität: 23.12.1996 EP 96120844
(43) Veröffentlichungstag der Anmeldung: 01.07.1998
(73) Patentinhaber: F. HOFFMANN-LA ROCHE AG, 4070 Basel (CH)
(72) Erfinder: Broger, Emil Albin, 4312 Magden (CH); Scalone, Michelangelo, 4127 Birsfelden (CH); Wehrli, Christof, 4108 Witterswil (CH)
(74) Vertreter: Kjellsaa-Berger, Hanny, Dr.

(56) Entgegenhaltungen:
- WILLOUGHBY C A ET AL: "CATALYTIC ASYMMETRIC HYDROGENATION OF IMINES WITH A CHIRAL TITANOCENE CATALYST: SCOPE AND LIMITATIONS" JOURNAL OF THE AMERICAN CHEMICAL SOCIETY,US,AMERICAN CHEMICAL SOCIETY, WASHINGTON, DC, Bd. 116, Nr. 20, 1. Januar 1994 (1994-01-01), Seiten 8952-8965, XP002046440 ISSN: 0002-7863
- W. OPPOLZER ET AL.: "Chiral toluene-2,alpha-sultam auxiliaries: Preparation and structure mof enantiomerically pure (R)- and (S)-ethyl-2,1'-sultam" TETRAHEDRON LETTERS, Bd. 31, Nr. 29, 1990, Seiten 4117-20, XP000864364
- Y. NG CHEONG CHAN ET AL.: "Iridium (III) Hydride Complexes for the Catalytic Enantioselective Hydrogenation of Imines" JOURNAL OF THE AMERICAN CHEMICAL SOCIETY, Bd. 112, 1990, Seiten 9400-9401, XP000864363
- W. R. CULLEN ET AL.: "Asymmetric homogeneous hydrogenation of imines using rhodium-phosphine systems" JOURNAL OF MOLECULAR CATALYSIS, Bd. 62, 1990, Seiten 243-53, XP000864377
- KITAMURA M ET AL: "GENERAL ASYMMETRIC SYNTHESIS OF ISOQUINOLINE ALKALOIDS. ENANTIOSELECTIVE HYDROGENATION OF ENAMIDES CATALYZED BY BINAP-RUTHENIUM(II) COMPLEXES" JOURNAL OF ORGANIC CHEMISTRY,US,AMERICAN CHEMICAL SOCIETY. EASTON, Bd. 59, Nr. 2, 1. Januar 1994 (1994-01-01), Seiten 297-310, XP002050844 ISSN: 0022-3263
- KITAMURA M ET AL: "GENERAL ASYMMETRIC SYNTHESIS OF BENZOMORPHANS AND MORPHINANS VIA ENANTIOSELECTIVE HYDROGENATION" TETRAHEDRON LETTERS,NL,ELSEVIER SCIENCE PUBLISHERS, AMSTERDAM, Bd. 28, Nr. 41, 1. Januar 1987 (1987-01-01), Seiten 4829-4832, XP002050843 ISSN: 0040-4039
- R. IMWINKELRIED: "Catalytic Asymmetric Hydrogenation in the Manufacture of d-Biotin and Dextromethorphan" CHIMIA, Bd. 51, Nr. 6, 1997, Seiten 300-302, XP000864390

## Beschreibung

Die vorliegende Erfindung betrifft ein neues, katalytisches Verfahren zur Herstellung von optisch aktiven Verbindungen der allgemeinen Formel 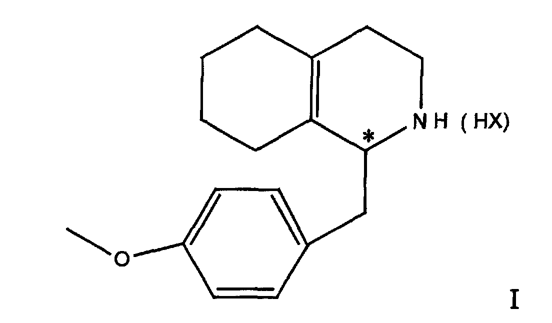 worin
HX eine Mineralsäure aus der Gruppe von HBF₄, H₂SO₄, HPF₆, HBr, HI, HCl, HSbF₆, HClO₄, oder starke organische Säuren aus der Gruppe von C₁₋₈-AlkylSO₃H, Pikrinsäure, Ameisensäure, Essigsäure,
Propionsäure, eine Arylcarbonsäure wie z.B. Benzoesäure, oder eine Dicarbonsäure wie z.B. Oxalsäure, Bernsteinsäure, Maleinsäure oder Phthalsäure bedeutet,
ausgehend von einer Verbindung der allgemeinen Formel 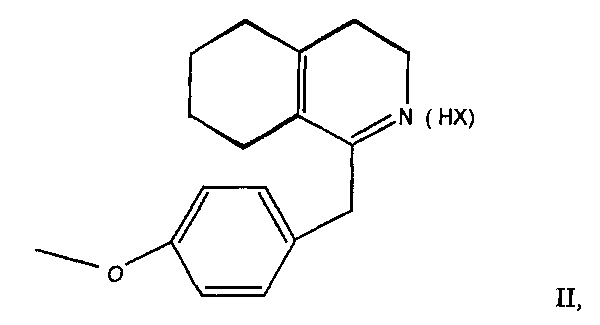 worin HX obige Bedeutung hat.

Die Verbindungen der Formel I in Form ihrer freien Basen sind an sich bekannte und wertvolle Zwischenprodukte für pharmakologisch verwendbare Endprodukte wie z.B. Dextromethorphan.

Die asymmetrische Hydrierung von Kohlenstoff-Stickstoff-Doppelbindungen in Iminen ist bekannt. Bisher wurden Rhodium-, Iridium- oder Titankatalysatoren (J. Mol. Catal. 1990, 62, 243; J. Am. Chem. Soc., 1994, 116, 8952 und J. Am. Chem. Soc., 1990, 112, 9400) verwendet. Dabei wurden die Imine immer als freie Base eingesetzt. Rutheniumkatalysatoren sind nur für speziell funktionalisierte Substrate bekannt (Tetrahedron Letters 1990, 31, 4117). J.Org.Chem Vol. 59, Nr. 2, 297 und Tetrahedron Letters 1987, 28, 4829-4832 berichten von der Herstellung der optisch aktiven Verbindungen der Formel I als freie Basen aus den entsprechenden freien Basen der Formel II über weitere Verfahrensschritte (Formylierung).

Problematisch ist die Hydrierung der Verbindung der Formel II als freie Base, da sie in dieser Form instabil ist und zur entsprechenden 1-(4-Methoxybenzyl)-5,6,7,8-tetrahydro-isochinolin (Tetrabase) als auch zum rac-1-(4-Methoxy-benzyl)-1,2,3,4.5,6,7,8-octahydro-isochinolin (rac-Octabase) disproportioniert. Ausserdem weisen die bekannten Verfahren wie z.B. die Herstellung von Dextromethorphan den Nachteil der Racematspaltung auf, da die bekannte Hydrierung von einer Verbindung der Formel II in Gegenwart von einem heterogenen Katalysator zu einem Racemat führt, das aufgespalten werden muss, da nur das (S)-Enantiomer der Formel I verwendet wird. Im weiteren muss das unerwünschte R-Enantiomer der Verbindung der Formel I über mehrere Reaktionsschritte aufgearbeitet, racemisiert und recyclisiert werden.

Aufgabe der vorliegenden Erfindung ist es, ein neues Verfahren zur Verfügung zu stellen, dass gezielt die asymmetrische Hydrierung von En-Iminen in Form ihrer Salze ermöglicht, ohne dass die oben beschriebenen Nachteile auftreten.

Das erfindungsgemässe Verfahren ist dadurch gekennzeichnet, dass eine Verbindung der allgemeinen Formel 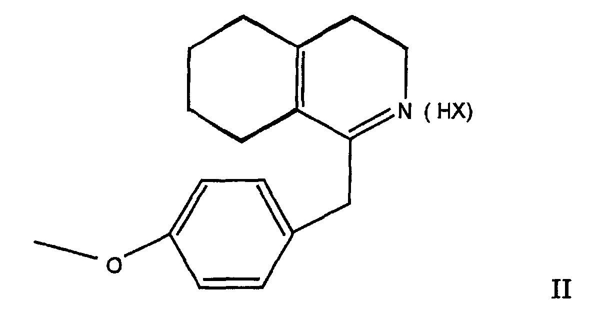 in der HX die obige Bedeutung hat,
in Gegenwart eines optisch aktiven, kationischen, anionischen oder neutralen Metall-Diphosphin-Komplexes der folgenden Formeln

[Ir(Y)(Lₙ)]⁺A⁻ III-c

[Ir(Y)(Lₙ)B] III-d

([Ir(Y)(B)₄])ₒ⁻M^{r+} III-e

[IrH(Y)(B)₂]₂ III-f

[Ir(Y)(B)₃]₂ III-g

[Ir(B)₃(Y)] III-h

asymmetrisch hydriert wird, wobei
- L: einen neutralen Liganden;
- A: ein Anion einer Sauerstoffsäure oder Komplexsäure;
- B: einen anionisch koordinierenden Liganden;
- n: 0, 1, 2;
- o: 1, 2;
- r: 1, 2;
- M⁺: ein Alkali- oder Erdalkali-metallkation oder tetrasubstituiertes Ammonium;
- Y: einen chiralen Diphosphinliganden gemäss den Formeln 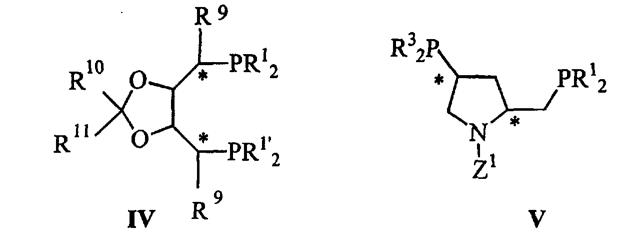
- R¹, R^{1'}: Aryl, Heteroaryl oder zusammen mit dem Phosphoratom ein 9-Dibenzophospholyl, wobei die Reste R¹ und R^{1'} gleich oder verschieden sein können;
- R⁹, R¹⁰, R¹¹: unabhängig voneinander Wasserstoff, C₁₋₈-Alkyl, C₁₋₈-halogeniertes Alkyl, C₃₋₈-Cycloalkyl, Aryl, Aralkyl; und R¹⁰ und R¹¹ zusammen einen 5- bis 8-gliedrigen Ring bilden können;
- R²: C₁₋₈-Alkyl oder C₃₋₈-Cycloalkyl, Aryl oder Aralkyl, oder zwei R² zusammen im gleichen Molekül einen 5- bis 8-gliederigen Ring bilden;
- R³: C₁₋₈-Alkyl, Heteroaryl, Aryl, C₃₋₈-Cycloalkyl, Aralkyl und
- Z¹: Wasserstoff, C₁₋₈-Alkyl, Aralkyl, -CO₂R², -CON(R²)₂, -SO₂R¹⁰, -PO(R¹⁰)₂ oder -COR³ bedeuten.

Die Verbindungen der Formel I und II, worin HX HBF4, H₂SO₄, HPF₆, HBr, HI, HSbF₆, HClO₄, oder starke organische Säuren aus der Gruppe von C₁₋₈-AlkylSO₃H, Pikrinsäure, Ameisensäure, eine Niederalkyl- oder Arylcarbonsäure wie z.B. Essigsäure, Propionsäure oder Benzoesäure, oder eine Dicarbonsäure wie z.B. Bernsteinsäure, Maleinsäure oder Phthalsäure bedeutet, sind ebenfalls Gegenstand der Erfindung.

Die optisch aktiven Iridiumkomplexe III-a bis III-g können für sich synthetisiert oder in situ in Abwesenheit oder in Gegenwart der zu hydrierenden Verbindungen der Formel II aus den Komponenten hergestellt werden. Im Zusammenhang mit den Verbindungen der Formeln III-a bis III-g und IV und V besitzen die nachstehenden Definitionen der allgemeinen Ausdrücke ihre Gültigkeit unabhängig davon, ob die fraglichen Ausdrücke allein oder in Kombination erscheinen.

Der Ausdruck "neutraler Ligand" bedeutet im Rahmen der vorliegenden Erfindung leicht austauschbare Liganden wie Olefine, z.B. Ethylen, Propylen, Cycloocten, 1,5-Hexadien, Norbornadien, 1,5-Cyclooctadien, Benzol, Hexamethylbenzol, p-Cymol und dergleichen, Nitrile wie Acetonitril, Benzonitril, oder auch verwendete Lösemittel wie z.B. THF, Toluol usw. Falls mehr als ein solcher Ligand vorhanden ist, können diese auch voneinander verschieden sein.

Der Ausdruck "Halogenide" umfasst Fluor, Chlor, Brom und Iod in Form von Alkali-, Erdalkali- oder tetrasubstituierten Ammoniumverbindungen.

Der Ausdruck "anionisch koordinierender Ligand" umfasst z.B. Halogen, einen Carbonsäurerest, einen Sulfonatrest wie z.B. Tosylat oder Methansulphonat, ein 1,3-Diketonat wie z.B. Acetylacetonat, ein gegebenenfalls substituiertes Phenolat, Hydroxy, Nitrit, Cyanat, Rhodanid, Cyanid, Allyl und 2-Methylallyl.

Der Ausdruck "Sauerstoffsäure oder Komplexsäure" bedeutet im Rahmen im Rahmen der vorliegenden Erfindung Säuren aus der Gruppe von H₂SO₄, HClO₄, HBrO₄, HIO₄, HNO₃, H₃PO₄, H₃PO₃, CF₃SO₃H, C₆H₅SO₃H aufgeführt, sowie Halogenkomplexe mit den Elementen Bor, Phosphor, Arsen Antimon oder Bismut. Bevorzugte Vertreter sind HClO₄, CF₃SO₃H, HPF₆, HBF₄, HB(Ph)₄, HB(3,5-C₆H₃)₄, HSbF₆ und HAsF₆.

Der Ausdruck "C₁₋₈-Alkyl" bedeutet im Rahmen der vorliegenden Erfindung für alle alkylenthaltenden Systeme Kohlenwasserstoffe mit 1 bis 8 Kohlenstoffatomen, d.h. geradkettige oder verzweigte Alkygruppen wie beispielsweise Methyl, Ethyl, Propyl, isoPropyl, Butyl, isoButyl, tert.-Butyl, Pentyl, isoPentyl, Neopentyl, Hexyl, tert.Hexyl, Heptyl, isoHeptyl, Octyl, isoOctyl.

Der Ausdruck "C₁₋₈-Alkoxy" bedeutet eine Alkylgruppe wie sie weiter oben definiert worden ist, welche über ein Sauerstoffatom gebunden ist. Beispielhaft seien Methoxy, Ethoxy, Propoxy, Isopropoxy, Butoxy und dergleichen erwähnt.

Der Ausdruck "C₃₋₈-Cycloalkyl" bedeutet im Rahmen der vorliegenden Erfindung Cyclopropyl, Cyclobutyl, Cyclopentyl, Cyclohexyl, Cycloheptyl, Cyclooctyl.

Der Ausdruck "halogeniertes Alkyl" bedeutet im Rahmen der vorliegenden Erfindung Alkylgruppen mit einer variablen Anzahl von Halogenatomen, insbesondere Chlor oder Fluor, wobei es sich vorzugsweise um wenigstens ein Halogenatom oder aber perfluorierte oder perchlorierte Verbindungen, wie beispielsweise Trifluormethyl, Trichlormethyl, Pentafluorethyl und dergleichen, handelt.

Der Ausdruck "Aryl" bedeutet Phenylreste, welche sowohl unsubstituiert als auch in ortho-, meta- oder para-Stellung einfach oder mehrfach substituiert sein können. Als Substituenten kommen in Frage Phenyl, C₁₋₈-Alkyl- oder Alkoxygruppen, C₁-C₈-halogeniertes Alkyl, oder auch Di-C₁-C₈ Alkylamino, Diphenylamino, Dibenzylamino, Morpholino, Pyperidino, Pyrrolidino, Halogen, Trialkylsilyl z.B. Trimethylsilyl und dergleichen. Der Ausdruck kann zudem auch Naphthyl bedeuten.

Der Ausdruck "Aralkyl" bedeutet Gruppen, in denen der Arylrest die vorhergehende Bedeutung hat und der Alkylrest ebenfalls die oben angeführte Bedeutung hat. Beispielhaft sei Benzyl und dergleichen angeführt.

Der Ausdruck "Heteroaryl" bedeutet in der vorliegenden Erfindung fünf- oder sechsgliedrige Heteroaromaten, die zusätzlich ankondensierte aromatische Gruppen aufweisen, mit einem oder mehreren Heteroatomen aus der Gruppe von Stickstoff, Sauerstoff oder Schwefel. Beispielhaft seien für die fünfgliedrigen Heteroaromaten Pyrrol, Thiophen, Furan erwähnt. Aus der Gruppe der sechsgliedrigen Heteroaromaten sei beispielhaft das Pyridin angeführt. Die Heteroaromaten können wie die oben aufgeführten Aryle substituiert sein, wobei bei stickstoffhaltigen Heteroaromaten der Stickstoff zusätzlich mit Wasserstoff, Alkylgruppen oder Alkoxygruppen substituiert sein kann.

Die Liganden der Formeln IV und V sind an sich bekannte Verbindungen und können beispielsweise wie in SYNLETT 1992, 169 beschrieben, hergestellt werden.

Die erfindungsgemässe, asymmetrische Hydrierung von Verbindungen der allgemeinen Formel II zu Verbindungen der allgemeinen Formel I wird in geeigneten, unter den Reaktionsbedingungen inerten organischen Lösemitteln erfolgen. Als derartige Lösemittel kommen insbesondere in Betracht niedere Alkohole, wie Methanol, Ethanol, iso-Propanol; Ester; halogenierten Kohlenwasserstoffen, wie beispielsweise Methylenchlorid, Chloroform und dergleichen; Kohlenwasserstoffe aus der Gruppe von Toluol, Xylol und dergleichen; Ether wie beispielsweise tert.-Butylmethylether, Diethylether, Tetrahydrofuran, Dioxan, Furan oder Amide wie Dimethylformamid (DMF); Nitrile wie Acetonitril, Carbonsäuren wie Essigsäure, Sulfoxide wie Dimethylsulfoxid (DMSO). Ferner können Gemische dieser Lösemittel untereinander oder zusätzlich mit Wasser verwendet werden. Bevorzugte Lösemittel sind Kohlenwasserstoffe, Alkohole und Wasser bzw. Gemische davon. Ein besonders bevorzugtes Lösemittelgemisch besteht aus Alkohol und Kohlenwasserstoffen wie Toluol und Methanol. Ein besonders bevorzugtes Lösemittelgemisch aus drei Lösemitteln besteht aus Toluol, Methanol und Wasser.

Das erfindungsgemässe Verfahren wird vorzugsweise in Gegenwart eines Additives in Form von Basen ausgeführt. Bei den Basen handelt es sich um Verbindungen der Gruppe von Carbonsäuresalzen wie z.B. Natriumacetat, Natriumformiat und dergleichen, primären, sekundären und tertiären Aminen wie beispielsweise Diisopropylamin, Triethylamin, Diisopropylethylamin, sowie Diaminen des Typs Ethylendiamin, Tetramethylethylendiamin, Imide wie Succinimid, Phthalimid, Natriumalkylate wie beispielsweise Natriummethylat, und Natriumhydroxid. Als ein weiteres Additiv ist auch (R oder S)-1-(4-Methoxybenzyl)-1,2,3,4,5,6,7,8-octahydro-isochinolin zu nennen. Besonders bevorzugt werden tertiäre Amine wie Diisopropylamin und Triethylamin. Ebenfalls bevorzugte Additive sind Carbonate wie Natriumhydrogencarbonat, Natriumcarbonat, Kaliumhydrogencarbonat wie auch Dinatriumhydrogenphosphat und dergleichen.

Durch die Zugabe von Additiven werden sowohl die enantiomere Reinheit als auch die Ausbeuten entscheidend erhöht.

Die Mengen der eingesetzten Additive liegen im Bereich von 0,001 bis 100 Molequivalenten bezogen auf die Verbindungen gemäss der Formeln II bzw. III-a - III-g.

Wird als Additiv eine Base aus der oben genannten Gruppe verwendet, liegt deren Menge im Bereich von 0,001-100 Molequivalenten, bevorzugt im Bereich von 0,001-10 Molequivalenten und besonders bevorzugt im Bereich von 0,001-2 Molequivalenten bezogen auf die Verbindungen gemäss Formel II.

Die anionischen koordienierenden Liganden gemäss der oben ausgeführten Definition können dem Reaktionsgemisch zusätzlich in Mengen von 0,1-100 Molequivalenten, bevorzugt 0,5-50 Molequivalenten und besonders bevorzugt bevorzugt 1-10 Molequivalenten bezogen auf die Verbindungen der Formel III-a - III-g eingesetzt werden.

Eine spezielle Ausführungsform des Verfahrens zur asymmetrischen Hydrierung ist die Verwendung von flüssigem oder überkritischem Kohlendioxid als Lösemittel oder in Verbindung mit anderen der oben aufgeführten Lösemitteln. Bereits ein geringer prozentualer Anteil an Kohlendioxid in der Reaktionsmischung führt zu einer Ausbeuteerhöhung des Produktes wie den Beispielen 1.6 bis 1.8 und dem Beispiel 1.5 als Vergleichsversuch zu entnehmen ist.

Die Salze der Verbindungen der Formel II können in an sich bekannter Weise hergestellt werden, z.B. ausgehend vom bekannten Hydrochlorid der Formel II durch Umsalzen oder durch Säureaustausch. Dabei kann auf die Verwendung von Wasser und/oder chlorierten Lösemitteln verzichtet werden.

Die asymmetrische Hydrierung wird zweckmässig bei Temperaturen im Bereich von etwa 10°C bis etwa 200°C, vorzugsweise 10°C bis 100°C, und besonders bevorzugt in einem Bereich von 20°C bis 100°C, und einem Druck von etwa 1 bis 250 bar, bevorzugt 1 bis 180 bar und besonders bevorzugt 10 bis 90 bar, durchgeführt.

Das molare Verhältnis (S/C) zwischen den zu hydrierenden Verbindungen der Formel II und den Metallkomplexen, die als Katalysatoren gemäss den Formeln III-a bis III-g verwendet werden, liegt zweckmässig zwischen 20 bis 80 000, vorzugsweise zwischen 100 bis 50 000 und besonders bevorzugt zwischen 100 bis 30 000.

Für die asymmetrische Hydrierung von Verbindungen der Formel II werden Iridium-Komplexe mit optisch aktiven Diphosphinliganden der Formeln IV und V verwendet.

Beispiele besonders bevorzugter Liganden der Formeln IV und V sind
(2R,3R)-O-Isopropyliden-2,3-dihydroxy-1,4-bis-[bis-(4-methoxy-3,5-dimethyl-phenyl)phosphino]butan
tert-Butyl-(2S, 4S)-4-(di-m-Tolylphosphino)-2-[(di-m-tolylphosphino)-methyl]-1-pyrrolidincarboxylat
(2R,3R)-O-Isopropyliden-2,3-dihydroxy-1,4-bis- bis-(4-methoxy-3,5-diisopropyl-phenyl)phosphino butan
(2R,3R)-O-Isopropyliden-2,3-dihydroxy-1,4-bis-bis-(3,5-ditert-butylphenyl)phosphino butan
(2R,3R)-O-Isopropyliden-2,3-dihydroxy-1,4-bis-[bis-(3,5-di-N-morpholino-phenyl)phosphino]butan
(2R,3R)-O-Isopropyliden-2,3-dihydroxy-1,4-bis-[bis-(3,4,5-trimethoxyphenyl)phosphino]butan
(2R,3R)-O-Isopropyliden-2,3-dihydroxy-1,4-bis-[bis-(2-naphthyl)-phosphino]butan
(2R,3R)-O-Isopropyliden-2,3-dihydroxy-1-(dicyclohexylphosphino)-4-[bis-(4-methoxy-3,5-ditert-butyl-phenyl)phosphino]butan
(2R,3R)-O-Isopropyliden-2,3-dihydroxy-1-(diphenylphosphino)-4-[bis-(4-methoxy-3,5-ditert-butyl-phenyl)phosphino]butan
(2R,3R)-O-Isopropyliden-2,3-dihydroxy-1-(dicyclohexylphosphino)-4-[bis-(3,5-ditert-butyl-phenyl)phosphino]butan
tert-Butyl-(2S,4S)-4-[bis-(4-methoxy-3,5-dimethyl-phenyl)phosphino]-2-[bis-(4-methoxy-3,5-dimethyl-phenylphosphino)methyl]-1-pyrrolidincarboxylat
tert-Butyl-(2S,4S)-4-[bis-(3,5-ditert-butyl-phenyl)phosphino]-2-[bis-(3,5-ditert-butyl-phenylphosphino)methyl]-1-pyrrolidincarboxylat
tert-Butyl-(2S,4S)-4-[diphenyl)phosphino]-2-[bis-(3,5-ditert-butyl-phenylphosphino)methyl]-1-pyrrolidincarboxylat
tert-Butyl-(2S,4S)-4-[bis-(4-methoxy-3,5-ditert-butyl-phenyl)phosphino]-2-[bis-(4-methoxy-3,5-ditert-butyl-phenylphosphino)methyl]-1-pyrrolidincarboxylat

Die folgenden Beispiele dienen der Erläuterung der Erfindung und stellen keinerlei Einschränkung hiervon dar. In diesen Beispielen haben die verwendeten Abkürzungen folgende Bedeutung
- HPLC: Hochdruck-Flüssigchromatographie
- RT: Raumtemperatur
- HV: Hochvakuum
- GC: Gaschromatographie
- e.e.: Enantiomerer Ueberschuss
- (R,R)-MOD-DIOP:: (4R,5R)-O-Isopropyliden-2,3-dihydroxy-1,4-bis-[bis-(4-methoxy-3,5-dimethylphenyl)phosphino]butan
- (S,S)-mTol-BPPM:: tert-Butyl-(2S,4S)-4-(di-m-Tolylphosphino)-2-[(di-m-tolylphosphino)methyl]-1-pyrrolidincarboxylat
- (R,R)-3,5-tBu,4-Me-O-DIOP:: (2R,3R)-O-Isopropyliden-2,3-dihydroxy-1,4-bis-[bis-(4-methoxy-3,5-ditert-butylphenyl)phosphino]butan
- (R,R)-3,5-MOR-DIOP:: (2R,3R)-O-Isopropyliden-2,3-dihydroxy-1,4-bis-[bis-(3,5-di-N-morpholinophenyl)phosphino]butan
- (R,R)-3,4,5-MeO-DIOP:: (2R,3R)-O-Isopropyliden-2,3-dihydroxy-1,4-bis-[bis-(3,4,5-trimethoxy-phenyl)phosphino]butan
- (R,R)-2-Naphthyl-DIOP:: (2R,3R)-O-Isopropyliden-2,3-dihydroxy-1,4-bis-[bis-(2-naphthyl)phosphino]butan
- (R,R)-(Cy)₂(3,5-tBu,4-MeO)₂-DIOP:: (2R,3R)-O-Isopropyliden-2,3-dihydroxy-1-(dicyclohexylphospino)-4-[bis-(4-methoxy-3,5-ditert-butyl-phenyl)phosphino]butan
- (R,R)-(3,5-tBu,4-MeO)₂-DIOP:: (2R,3R)-O-Isopropyliden-2,3-dihydroxy-1-(diphenylphosphino)-4-[bis-(4-methoxy-3,5-ditert-butyl-phenyl)phosphino]butan
- (R,R)-(Cy)₂(3,5-tBu)₂-DIOP:: (2R,3R)-O-Isopropyliden-2,3-dihydroxy-1-(dicyclohexylphosphino)-4-[bis-(3,5-ditert-butyl-phenyl)phosphino]butan
- (S,S)-MOD-BPPM:: tert-Butyl-(2S,4S)-4-[bis-(4-methoxy-3,5-dimethyl-phenyl)phosphino]-2-[bis-(4-methoxy-3,5-dimethyl-phenylphosphino)methyl]-1-pyrrolidincarboxylat
- (S,S)-3,5-tBu-BPPM:: tert-Butyl-(2S,4S)-4-[bis-(3,5-ditert-butylphenyl)phosphino]-2-[bis-(3,5-ditertbutyl-phenylphosphino)methyl]-1-pyrrolidincarboxylat
- (S,S)-(3,5-tBu)2(Ph)2-BPPM:: tert-Butyl-(2S,4S)-4-[diphenyl)phosphino]-2-[bis-(3,5-ditert-butyl-phenylphosphino)methyl]-1-pyrrolidin-carboxylat
- (S,S)-3,5-tBu,4-MeO-BPPM:: tert-Butyl-(2S,4S)-4-[bis-(4-methoxy-3,5-ditert-butyl-phenyl)phosphino]-2-[bis-(4-methoxy-3,5-ditert-butyl-phenylphosphino)methyl]-1-pyrrolidincarboxylat
- Hexabase:: 1-(4-Methoxybenzyl)-3,4,5,6,7,8-hexahydro-isochinolin
- (S)-Octabase:: (S)-1-[4-Methoxy-benzyl]-1,2,3,4,5,6,7,8-octahydro-isochinolin

Alle Temperaturen sind in Grad Celsius angegeben.

### Beispiel 1

In einer Glove Box (02-Gehalt < 1ppm) wurden in einem 35 ml-Autoklaven mit Glaseinsatz 13.4 mg [IrCl(COD)]₂ (0.020 mmol) und 32.2 mg (R,R)-MOD-DIOP (0.044 mmol) als chiraler Ligand in 4 ml Methanol gelöst. Nach Zugabe von 59.1 mg Bu₄N⁺I⁻ (0.16 mmol) und Rühren während 30 min. wurden dieser Katalysatorlösung 0.343g 1-(4-Methoxybenzyl)-3,4,5,6,7,8-hexahydro-isochinolin tetrafluoroborat (1.0 mmol) und 4 ml Toluol zugegeben. Dann wurde der Autoklav verschlossen und die Hydrierung unter Rühren bei 25° und einem Druck von 100 bar Wasserstoff während 44 h durchgeführt. Die gelbe Hydrierlösung wurde am Rotationsverdampfer bei 40°/20 mbar eingedampft. Bei einem vollständigen Umsatz bestand der Rückstand gemäss HPLC- (Säule: ChiralPAK AD, Eluens: 10% Ethanol und 0.2% Triethylamin in Hexan) und GC- (Als Amid der (-)-Camphansäure, Säule: OV-240 OH 15m) Analyse aus 90% (S)-Octabase mit einem e.e. von 61%.

### Beispiele 1.1-1.10

In zu Beispiel 1 analoger Weise wurde die Hydrierung mit den in der Tabelle 1 aufgeführten chiralen Liganden durchgeführt.

**Tabelle 1**

| Beisp. | Chiraler Ligand | % Selekt. zu Octabase | % e.e. (Config.) |
|---|---|---|---|
| 1.1 | *(R,R)*-MOD-DIOP (a) | 94 | 60 (S) |
| 1.2 | *(R,R)*-MOD-DIOP (c) | 85 | 51 (S) |
| 1.3 | " (d) | 91 | 57 (S) |
| 1.4 | " (e) | 65 | 61 (S) |
| 1.5 | " (f) | 95 | 58 (S) |
| 1.6 | " (g) | 97 | 63(S) |
| 1.7 | " (h) | 53 | 32(S) |
| 1.8 | " (i) | 55 | 28(S) |
| 1.9 | (R,R)-3,5-tBu-DIOP | 75 | 84(S) |
| 1.10 | (R,R)-3,5-iProp-4-MeO-DIOP | 69 | 81(S) |

| | | | |
|---|---|---|---|
| a) Zusatz von 0.04 mmol Phthalimid anstelle von Bu₄N⁺I⁻; | | | |
| c) Zusatz von 1.0 mmol Et3N; | | | |
| d) Zusatz von 1.0 mmol NaOAc; | | | |
| e) THF als Lösemittel; | | | |
| f) DMF als Lösemittel. | | | |
| g) Lösemittel: MeOH4 ml/Toluol 4ml/ CO₂ 1,3g; | | | |
| h) Lösemittel: THF 1ml / CO₂ 12,0 g 45°C; | | | |
| i) Lösemittel 12,1 g CO₂, 45°C. | | | |

### Beispiele 2a.1-2a.17

Für die Beispiele in Tabelle 2a wird Beispiel 2a.1 im Detail beschrieben. Die Beispiele 2a.2 bis 2a.17 wurden analog durchgeführt.

In einer Glove-Box wurden in einem 185 ml-Autoklaven 1.01 mg [IrCl(COD)]₂ (0.0015 mmol) und 3.5 mg (R,R)-3,5-tBu-DIOP (0.0033 mmol) als chiraler Ligand in 18 ml Methanol gelöst. Nach Zugabe von 4.3 mg Bu₄N⁺I⁻ (0.012 mmol) und Rühren während 30 min wurden dieser Katalysatorlösung 2.12 g Hexabase-Hydrogensulfat (6.0 mmol), 77.7 mg Diisopropylethylamin (0.6 mmol) und 18 ml Toluol zugegeben. Dann wurde der Autoklav verschlossen und die Hydrierung unter Rühren bei 80° und einem Druck von 40 bar Wasserstoff während 4-6 h durchgeführt. Die gelbe Hydrierlösung wurde am Rotationsverdampfer eingedampft. Bei einem vollständigen Umsatz bestand der Rückstand gemäss HPLC and GC aus 95% (S)-Octabase mit einem ee von 80%.

In analoger Weise werden die Beispiele

**Tabelle 2a**

| Beisp. | Chiraler Ligand | Selekt. Zu Octabase | % e.e. (Config) |
|---|---|---|---|
| 2a.1 | (R,R)-3,5-tBu,4-MeO-DIOP | 95 | 80 (S) |
| 2a.2 | (R,R)-3,5-tBu-DIOP | 97 | 80 (S) |
| 2a.3 | (R,R)-3,5-Ipr,4-MeO-DIOP | 94 | 80 (S) |
| 2a.4 | (R,R)-MOD-DIOP | 95 | 52 (S) |
| 2a.5 | (R,R)-2-Naphtyl-DIOP | 98 | 24 (S) |
| 2a.6 | (R,R)-3,5-MOR-DIOP | 94 | 45 (S) |
| 2a.7 | (R,R)-3,4,5-MeO-DIOP | 90 | 39 (S) |
| 2a.8 | (R,R)-DIOP | 94 | 27 (S) |
| 2a.9 | (R,R)-(Cy)₂(3,5-tBu,4-MeO)₂-DIOP | 96 | 61 (S) |
| 2a.10 | (R,R)-(3,5-tBu,4-MeO)₂-DIOP | 98 | 46 (S) |
| 2a.11 | (R,R)-(Cy)₂(3,5-tBu)₂-DIOP | 98 | 65 (S) |
| 2a.12 | (S,S)-3,5-Me,4MeO-BPPM | 95 | 46 (S) |
| 2a.13 | (S,S)-mTol-BPPM | 94 | 40 (S) |
| 2a.14 | (S,S)-BPPM | 95 | 30 (S) |
| 2a.15 | (S,S)-3,5-tBu-BPPM | 93 | 29 (S) |
| 2a.16 | (S,S)-(3,5-tBu,4-MeO)₂(Ph)₂-BPPM | 99 | 21 (S) |
| 2a.17 | (S,S)-3,5-tBu,4-MeO-BPPM | 98 | 26 (S) |

### Beispiele 2b.1-2b.13

In zu Beispiel 1 analoger Weise wurde die Hydrierung von Hexabase-Hydrogensulfat in den in der Tabelle 2b aufgeführten Lösungsmitteln durchgeführt (Zusatz von 0.1 mmol iPr₂NEt als Base, Umsatz 40-100%)

**Tabelle 2b**

| Beisp. | Lsm**1** | Lsm**2** | Selekt. Zu Octabase | % e.e. (Konfig) |
|---|---|---|---|---|
| 2b.1 | Toluol (4ml) | MeOH (4ml) | 84 | 89 |
| 2b.2 | Toluol (4ml) | MeOH (4ml) H₂O (0.2ml) | 72 | 86 |
| 2b.3 | MeOH (8ml) | | 77 | 84 |
| 2b.4^{a)} | MeOH (3ml) | CO₂ (6g) | 80 | 78 |
| 2b.5 | 2-BuOH (8ml) | | 86 | 67 |
| 2b.6 | THF (8ml) | H₂O (0.2ml) | 81 | 66 |
| 2b.7 | MeOH (4ml) | CH₂Cl₂ (4ml) | 91 | 65 |
| 2b.8 | THF (8ml) | | 90 | 63 |
| 2b.9 | THF (4ml) | CH₂Cl₂ (4ml) | 75 | 56 |
| 2b.10 | AcOEt (8ml) | | 76 | 54 |
| 2b.11 | IprOH (8ml) | | 49 | 53 |
| 2b.12 | Toluol (4ml) | IprOH (4ml) | 57 | 49 |
| 2b.13 | Toluol (8ml) | | 56 | 47 |

| | | | | |
|---|---|---|---|---|
| ^{a)} 60°, 65h, 220 bar Gesamtdruck | | | | |

### Beispiele 3.1-3.3

In zu Beispiel 1 analoger Weise wurde die Hydrierung von 1-(4-5 Methoxybenzyl)-3,4,5,6,7,8-hexahydro-isochinolin-hexafluorophosphat mit den in der Tabelle 4 aufgeführten chiralen Liganden durchgeführt.

**Tabelle 3**

| Beisp. | Katal. | Chiraler Ligand | % Selekt. zu Octabase | % e.e. (Config.) |
|---|---|---|---|---|
| 3.1 | [Ir] | (R,R)-MOD-DIOP | 90 | 60 (S) |
| 3.2 | " | (R,R)-MOD-DIOP (a) | 81 | 48 (S) |
| 3.3 | " | (R,R)-MOD-DIOP (b) | 62 | 62 (S) |

| | | | | |
|---|---|---|---|---|
| a) Kein Zusatz von Bu₄N⁺I⁻; | | | | |
| b) THF als Lösungsmittel. | | | | |

### Beispiel 4

In einer Glove Box (O₂-Gehalt < 1ppm) wurden in einem 35 ml-Autoklaven mit Glaseinsatz 9.9 mg [Ir(COD)₂]BF₄ (0.020 mmol) und 16.1 mg (R,R)-MOD-DIOP (0.022 mmol) als chiraler Ligand in 4 ml THF gelöst und 30 min gerührt. Nach Zugabe von 29.5 mg Bu₄N⁺I⁻ (0.08 mmol) und 15 min Rühren wurden 0.34 g 1-(4-Methoxybenzyl)-3,4,5,6,7,8-hexahydro-isochinolin bisulphat (1.0 mmol), 4 ml THF und 0.2 ml Wasser zugegeben, der Autoklav verschlossen und die Hydrierung unter Rühren bei 25° und einem Druck von 100 bar Wasserstoff während 20 h durchgeführt. Die gelbe Hydrierlösung wurde am Rotationsverdampfer bei 40°/20 mbar eingedampft. Bei einem vollständigen Umsatz bestand der Rückstand gemäss HPLC- and GC-Analyse aus 67% (S)-Octabase mit einem e.e. von 71%.

### Beispiel 5

Der Versuch wurde in zu Beispiel 4 analoger Weise durchgeführt; jedoch wurden 25.8 mg Bu₄N⁺Br⁻ (0.08 mol) anstelle von Bu₄N⁺I⁻ verwendet. Bei einem vollständigen Umsatz bestand der Rückstand gemäss HPLC- and GC-Analyse aus 65% (S)-Octabase mit einem e.e. von 66%.

### Beispiel 6

Der Versuch wurde in zu Beispiel 4 analoger Weise durchgeführt, Jedoch wurde kein Tetrabutylammoniumjodid zugesetzt. Bei einem vollständigen Umsatz bestand der Rückstand gemäss HPLC- und GC-Analyse aus 87 % (S)-Octabase, e.e. = 56 %.

### Beispiel 7

In einem 350 ml Vierhalssulfierkolben wurden 27.34 g (100 mMol) N-(2-Cyclohex-1-enylethyl)-2-(4-methoxyphenyl)acetamid in 30 ml Toluol vorgelegt. Bei 80°C wurde in einer Inertgasatmosphäre mit einer Motorkolbenbürette innert 60 Minuten 4.73 ml destilliertes Phosphoroxychlorid (51 mMol) zudosiert. Die Lösung wurde: 1h bei 80°C, danach 2h bei 90°C und schliesslich noch 1 h bei 100°C nachreagieren gelassen. Die Reaktionsmischung enthielt das gelöste rohe Bischler-Napieralsky Reaktionsprodukt 1-(4-Methoxybenzyl)-3,4,5,6,7,8-hexahydro-isochinolin der Formel II in Form eines Salzgemisches bestehend aus HCl und div. Phosphorsäuren.

### Beispiel 8

Zu der in Beispiel 7 hergestellten Reaktionsmischung in Toluol wurden bei 20°C in einer Inertgasatmosphäre innert 30 min. 20.2 g Schwefelsäure 97% (200mMol) zugetropft, wobei gasförmige Salzsäure entwich. Das Toluol wurde anschliessend im Wasserstrahlvakuum abdestilliert. Der Rückstand wurde in 250 ml Isopropanol gelöst. Nach 18 h kristallisieren bei 0°C wurde das Kristallisat abgenutscht und mit eiskaltem Isopropanol nachgewaschen. Nach dem Trocknen im Wasserstrahlvakuum wurden erhalten: 32.4g 1-(4-Methoxybenzyl)-3,4,5,6,7,8-hexahydro-isochinolin hydrogensulfat, Smp = 186-7°C.

| Massenanalyse: | | | | | |
|---|---|---|---|---|---|
| | C | H | N | S | |
| Ber | 57.77 | 6.56 | 3.96 | 9.07 | % |
| Gef | 57.39 | 6.49 | 3.97 | 9.14 | % |

### Beispiel 9

Analog zu Beispiel 8 wurde 1-(4-Methoxybenzyl)-3,4,5,6,7,8-hexahydroisochinolinperchlorat isoliert mit Smp. 128-131°.

### Beispiel 10

Zu der in Beispiel 7 hergestellten Reaktionsmischung in Toluol wurde bei RT in einer Inertgasatmosphäre nacheinander 160 ml Essigester und eine Lösung von 10.98 g Natriumfluoroborat (100 mMol) gelöst in 40 ml Wasser gegeben. Nach 10 Min. Rühren wurde die Wasserphase abgetrennt und die organische Phase im Vakuum schonend eingeengt. Der Rückstand wurde mit 2x 100ml Essigester im Vakuum azeotropisch getrocknet und anschliessend in einer Inertgasatmosphäre in 50 ml Essigester gelöst. Nach 8 h kristallisieren bei 0°C wurde das Produkt abgenutscht und mit 25 ml Essigester nachgewaschen. Nach dem Trocknen bei 40° im Wasserstrahlvakuum erhielt man 28.1 g 1-(4-Methoxybenzoyl)-3,4,5,6,7,8-hexahydro-isochinolin tetrafluoroborat, Smp = 97-8°C.

### Beispiel 11

Analog zu Bespiel 10 wurde 1-(4-Methoxybenzyl)-3,4,5,6,7,8-hexahydroisochinolinhexafluorophosphat isoliert, Smp =161-162°C.

### Beispiel 12

Analog zu Beispiel 10 wurde 1-(4-Methoxybenzyl)-3,4,5,6,7,8-hexahydroisochinolinpikrat isoliert, Smp. 131-134°C.

### Beispiel 13

Analog zu Beispiel 10 wurde 1-(4-Methoxybenzyl)-3,4,5,6,7,8-hexahydroisochinolinphthalat isoliert. Smp. 116-120°C.

### Beispiel 14

Ein 2 l-Rührautoklav wurde unter Argonüberleitung mit 148.45 g Hexabase Hydrogensulfat (420 mol), 467 ml Methanol, 507 ml Toluol und 4.25 ml Triethylamin beladen und verschlossen. Nach dem Austausch der Luftatmosphäre mit Wasserstoff wurde eine Katalysatorlösung bestehend aus 14.1 mg [IrCl(COD)]₂ (0.021 mmol), 43.8 mg (R,R)-3,5-tBu-DIOP (0.0462 mmol), 62.1 mg Bu₄N⁺I⁻ (0.168 mmol) in 40 ml Methanol aus einem Katalysatorzugabegefäss einlaufen gelassen. Die Hydrierung wurde bei 80° und unter einem Wasserstoffdruck von 30 bar während 15 h durchgeführt. Dann wurde der Autoklav entleert und die Hydrierlösung eingedampft. Der Rückstand wurde in Wasser gelöst, mit 200 ml tert.Butyl-methylether extrahiert und dann mit einem Ueberschuss einer Natronlaugelösung und mit Hexan versetzt. Die Hexanphase wurde abgetrennt, neutral gewaschen und am Rotationsverdampfer eingedampft. Bei einem vollständigen Umsatz bestand der Rückstand (104.9 g) gemäss HPLC und GC aus 93.0% (S)-Octabase mit einem e.e. von 78.1%. Die ee-Anreicherung wurde durch Kristallisation des 1:1 Salzes der (S)-Octabase mit L-(+)-Mandelsäure aus Wasser erzielt. Dieses leicht beige kristallines Material (138.2 g, 81% Ausbeute) wurde mit einem Ueberschuss einer Natronlaugelösung und mit Hexan versetzt. Die Hexanphase wurde neutral gewaschen und eingedampft. Die erhaltene (S)-Octabase (86.2 g, 80% Ausbeute) wies einen Gehalt von 98.0% und einen ee von 98.6% auf.

### Beispiel 15

In einer Glove-Box wurden in einem 35 ml Autoklaven mit Glaseinsatz 6.72 mg [IrCl(COD)]2 (0.010 mmol) und 20.8 mg (R,R)-3,5-tBu-DIOP (0.022 mmol) als chiraler Ligand in einem Gemisch bestehend aus 4 ml Methanol und 2 ml Toluol gelöst. Nach Zugabe von 25.8 mg Bu₄N⁺I⁻ (0.08 mmol) und Rühren während 30 min wurden dieser Katalysatorlösung 345 mg Hexabase-Oxald(1.0 mmol) und 2 ml Toluol zugegeben. Dann wurde der Autoklav verschlossen und die Hydrierung unter Rühren bei 25° und einem Druck von 100 bar Wasserstoff während 18 h durchgeführt. Die gelbe Hydrierlösung wurde am Rotationsverdampfer eingedampft. Bei einem Umsatz von 99 % bestand der Rückstand gemäss HPLC and GC aus 65 % (S)-Octabase mit einem ee von 70 %.

### Beispiel 16

In zu Beispiel 15 analoger Weise, jedoch in Gegenwart von Ethyl-di-isopropylamin wurde die Hydrierung von 345 mg Hexabase-Oxalat (1.0 mmol) durchgeführt. Bei einem Umsatz von 92 % bestand der Rückstand gemäss HPLC and GC aus 60 % (S)-Octabase mit einem ee von 75 %.

### Beispiel 17

In zu Beispiel 15 analoger Weise, jedoch in Gegenwart von Ethyl-di-isopropylamin bei einer Temperatur von 80°C, wurde die Hydrierung von 345 mg Hexabase-Oxalat (1.0 mmol) durchgeführt. Bei einem Umsatz von 100 % bestand der Rückstand gemäss HPLC and GC aus 86 % (S)-Octabase mit einem ee von 68 %.

## Patentansprüche

1. Verfahren zur Herstellung von optisch aktiven R oder S 1-(4-Methoxybenzyl)-1,2,3,4,5,6,7,8-octahydro-isochinolin-Addukten der allgemeinen Formel 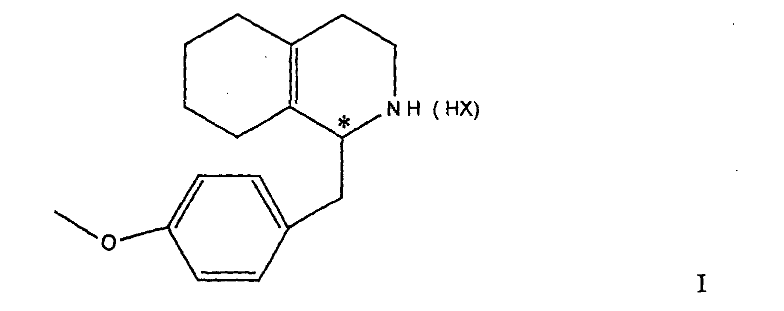 worin
HX eine Mineralsäure aus der Gruppe von HBF₄, H₂SO₄, HPF₆, HBr, HI, HCl, HSbF₆, HClO₄, starke organische Säuren aus der Gruppe von C₁₋₈-AlkylSO₃H, Pikrinsäure, Ameisensäure, Essigsäure, Propionsäure, eine Arylcarbonsäure oder eine Dicarbonsäure bedeutet,
**dadurch gekennzeichnet, dass** eine Verbindung der allgemeinen Formel 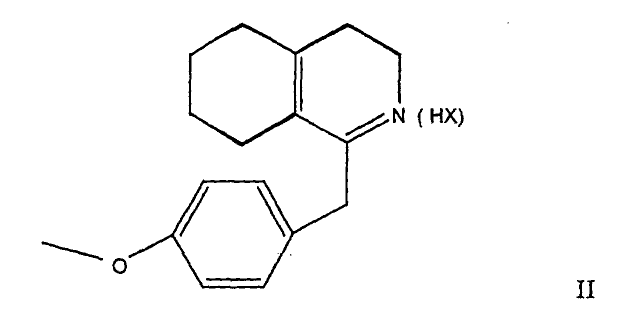 worin HX die oben genannte Bedeutung hat
in Gegenwart eines optisch aktiven, kationischen, anionischen oder neutralen Metall-Diphosphin-Komplexes der folgenden Formeln
[Ir(Y)(Lₙ)]⁺A⁻ III-c
[Ir(Y)(Lₙ)B] III-d
([Ir(Y)(B)₄])ₒ⁻M^{r+} III-e
[IrH(Y)(B)₂]₂ III-f
[Ir(Y)(B)₃]₂ III-g
[Ir(B)₃(Y)] III-h
asymmetrisch hydriert wird,
worin
L einen neutralen Liganden;
A ein Anion einer Sauerstoffsäure oder Komplexsäure;
B einen anionisch koordinierenden Liganden;
n 0, 1, 2;
o 1, 2;
r 1, 2;
M⁺ ein Alkali- oder Erdalkali-metallkation oder tetrasubstituiertes Ammonium;
Y einen chiralen Diphosphinliganden gemäss den Formeln 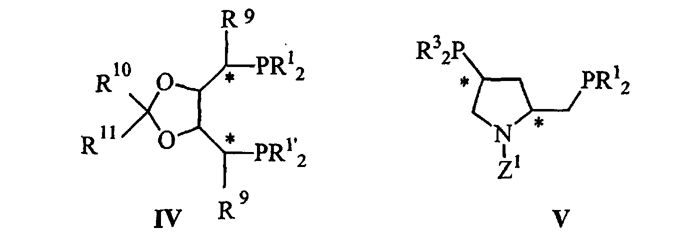
R¹, R^{1'} Aryl, Heteroaryl oder zusammen mit dem Phosphoratom ein 9-Dibenzophospholyl, wobei die Reste R¹ und R^{1'} gleich oder verschieden sein können;
R⁹, R¹⁰, R¹¹ unabhängig voneinander Wasserstoff, C₁₋₈-Alkyl, C₁₋₈-halogeniertes Alkyl, C₃₋₈-Cycloalkyl, Aryl, Aralkyl; und R¹⁰ und R¹¹ zusammen einen 5- bis 8-gliedrigen Ring bilden können;
R² C₁₋₈-Alkyl oder C₃₋₈-Cycloalkyl, Aryl oder Aralkyl, oder zwei R² zusammen im gleichen Molekül einen 5- bis 8-gliederigen Ring bilden;
R³ C₁₋₈-Alkyl, Heteroaryl, Aryl, C₃₋₈-Cycloalkyl, Aralkyl und
Z¹ Wasserstoff, C₁₋₈-Alkyl, Aralkyl, -CO₂R², -CON(R²)₂, -SO₂R¹⁰, -PO(R¹⁰)₂ oder -COR³ bedeuten.

2. Verfahren nach Anspruch 1, **dadurch gekennzeichnet, dass** die asymmetrische Hydrierung von Verbindungen der allgemeinen Formel II in Gegenwart einer Base als Additiv durchgeführt wird.

3. Verfahren zur Herstellung einer Verbindung der Formel I gemäss einem der Ansprüche 1 oder 2, **dadurch gekennzeichnet, dass** die asymmetrische Hydrierung in einem Temperaturbereich von 10°C bis 200°C, einem Druck von 1 bis 250 bar und einem Substrat zu Katalysator-Verhältnis von 20 - 80 000 erfolgt.

4. Verfahren nach einem der Ansprüche 1 oder 2, **dadurch gekennzeichnet, dass** als Additive Basen aus der Gruppe von Carbonsäuresalzen, Carbonate, primären, sekundären, tertiären Aminen und Imiden verwendet werden.

5. Verfahren nach einem der Ansprüche 2 oder 4, **dadurch gekennzeichnet, dass** als Basen sekundäre oder tertiäre Amine eingesetzt werden.

6. Verfahren nach einem der Ansprüche 1 oder 2, **dadurch gekennzeichnet, dass** die Menge des Additivs aus der Gruppe der Carbonsäure-salze, primären, sekundären und tertiären Aminen in einem Bereich von 0,001-100 Molequivalente, bezogen auf die Verbindung gemäss der Formel II beträgt.

7. Die Verbindungen
1-(4-Methoxy-benzyl)-3,4,5,6,7,8-hexahydro-isochinolin- hydrogensulfat,
1-(4-Methoxy-benzyl)-3,4,5,6,7,8-hexyhydro-isochinolin-hexafluorophosphat,
1-(4-Methoxy-benzyl)-3,4,5,6,7,8-hexyhydro-isochinolin-tetrafluorborat,
1-(4-Methoxy-benzyl)-3,4,5,6,7,8-hexahydro-isochinolin-phthalat,
1-(4-Methoxy-benzyl)-3,4,5,6,7,8-hexahydro-isochinolin-perchlorat.

## Claims

1. A process for the manufacture of optically active R or S 1-(4-methoxy-benzyl)-1,2,3,4,5,6,7,8-octahydro-isoquinoline adducts of the general formula 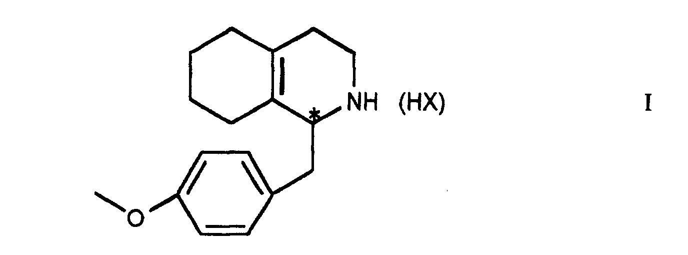 wherein
HX signifies a mineral acid from the group of HBF₄, H₂SO₄, HPF₆, HBr, HI, HCl, HSbF₆, HClO₄, strong organic acids from the group of C₁₋₈-alkylSO₃H, picric acid, formic acid, acetic acid, propionic acid, an arylcarboxylic acid or a dicarboxylic acid,
**characterized by** asymmetrically hydrogenating a compound of the general formula 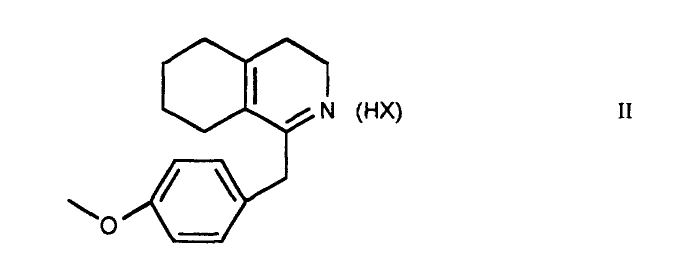 wherein HX has the significance given above,
in the presence of an optically active, cationic, anionic or neutral metal-diphosphine complex of the following formulae
[Ir(Y)(Lₙ)]⁺A⁻ III-c
[Ir(Y)(Lₙ)B] III-d
([Ir(Y)(B)₄])ₒ⁻M^{r+} III-e
[IrH(Y)(B)₂]₂ III-f
[Ir(Y)(B)₃]₂ III-g
[Ir(B)₃(Y)] III-h
wherein
L signifies a neutral ligand;
A signifies an anion of an oxygen acid or complex acid;
B signifies an anionic coordinating ligand;
n signifies 0, 1, 2;
o signifies 1, 2;
r signifies 1, 2;
M⁺ signifies an alkali or alkaline earth metal cation or tetrasubstituted ammonium;
Y signifies a chiral diphosphine ligand according to the formulae 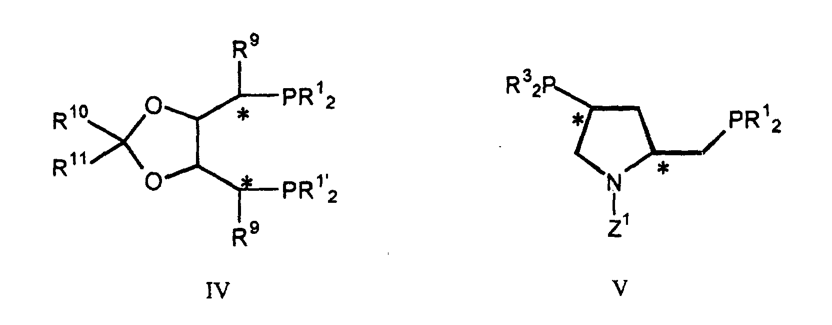
R¹, R^{1'} signify aryl, heteroaryl or together with the phosphorus atom a 9-dibenzophospholyl, whereby the residues R¹ and R^{1'} can be the same or different;
R⁹, R¹⁰, R¹¹ each independently signify hydrogen, C₁₋₈-alkyl, C₁₋₈-halogenated alkyl, C₃₋₈-cycloalkyl, aryl, aralkyl; and R¹⁰ and R¹¹ together can form a 5- to 8-membered ring;
R² signifies C₁₋₈-alkyl or C₃₋₈-cycloalkyl, aryl or aralkyl, or 2 R²'s together in the same molecule form a 5- to 8-membered ring;
R³ signifies C₁₋₈-alkyl, heteroaryl, aryl, C₃₋₈-cycloalkyl, aralkyl and
Z¹ signifies hydrogen, C₁₋₈-alkyl, aralkyl, -CO₂R², -CON(R²)₂, -SO₂R¹⁰, -PO(R¹⁰)₂ or -COR³.

2. A process according to claim 1, **characterized in that** the asymmetric hydrogenation of compounds of general formula II is carried out in the presence of a base as an additive.

3. A process for the manufacture of a compound of formula I in accordance with claim 1 or 2, **characterized in that** the asymmetric hydrogenation is effected in a temperature range of 10°C to 200°C, a pressure of 1 to 250 bar and a substrate to catalyst ratio of 20-80 000.

4. A process according to claim 1 or 2, **characterized in that** bases from the group of carboxylic acid salts, carbonates, primary, secondary, tertiary amines and imides are used as the additive.

5. A process according to claim 2 or 4, **characterized in that** secondary or tertiary amines are used as the bases.

6. A process according to claim 1 or 2, **characterized in that** the amount of the additive from the group of carboxylic acid salts, primary, secondary and tertiary amines is in a range of 0.001-100 mol equivalents based on the compound according to formula II.

7. The compounds
1-(4-methoxy-benzyl)-3,4,5,6,7,8-hexahydro-isoquinoline hydrogen sulphate,
1-(4-methoxy-benzyl)-3,4,5,6,7,8-hexyhydro-isoquinoline hexafluorophosphate,
1-(4-methoxy-benzyl)-3,4,5,6,7,8-hexyhydro-isoquinoline tetrafluoroborate,
1-(4-methoxy-benzyl)-3,4,5,6,7,8-hexahydro-isoquinoline phthalate,
1-(4-methoxy-benzyl)-3,4,5,6,7,8-hexahydro-isoquinoline perchlorate.

## Revendications

1. Procédé de préparation de produits d'addition optiquement actifs de R- ou de S-1-(4-méthoxybenzyl)-1,2,3,4,5,6,7,8-octahydro-isoquinoléine de formule générale 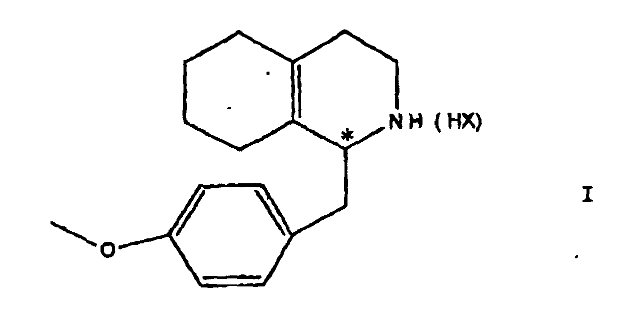 dans laquelle
HX est un acide minéral de l'ensemble comprenant HBF₄, H₂SO₄, HPF₆, HBr, HI, HCl, HSbF₆, HClO₄, un acide organique fort de l'ensemble comprenant (alkyle en C₁₋₈) - SO₃H, l'acide picrique, l'acide formique, l'acide acétique, l'acide propionique, un acide arylcarboxylique ou un acide diearboxylique,
**caractérisé en ce qu'**on soumet à une hydrogénation asymétrique un composé de formule générale 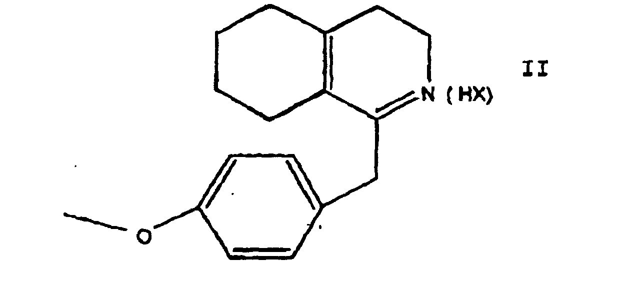 dans laquelle HX a les significations données ci-dessus,
en présence d'un complexe métal-diphosphine optiquement actif, cationique, anionique ou neutre, ayant les formules suivantes :
[Ir(Y) (Lₙ)]⁺A⁻ III-c
[Ir(Y) (Lₙ)B] III-d
[Ir(Y) (B)₄])ₒ⁻M^{r+} III-e
[IrH(Y) (B)₂]₂ III-f
[Ir(Y) (B)₃]₂ III-g
[Ir(B)₃(Y)] III-h
dans lesquelles
L est un ligand neutre ;
A est un anion d'un oxyacide ou d'un acide complexe ;
B est un ligand à coordination anionique ;
n vaut 0, 1 2 ;
o vaut 1, 2 ;
r vaut 1, 2 ;
M⁺ est un cation d'un métal alcalin ou alcalino-terreux, ou un ammonium cétraeubstitué ;
Y est un ligand diphosphine chiral ayant les formules 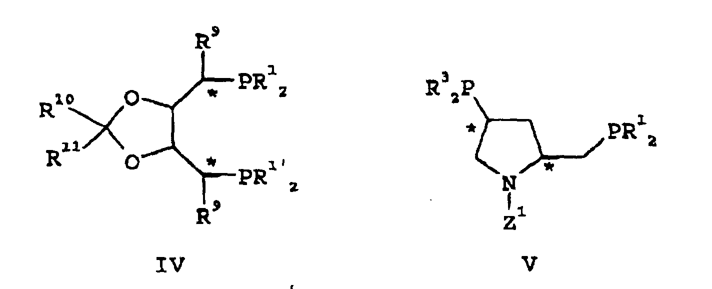
R¹, R^{1'} sont des radicaux aryle, hétéroaryle ou forment avec l'atome de phosphore un groupe 9-dibenzophospholyle, les radicaux R¹ et R^{1'} pouvant être identiques ou différents ;
R⁹, R¹⁰, R¹¹ représentent chacun indépendamment des autres un atome d'hydrogène, un groupe alkyle en C₁₋₈, alkyle en C₁₋₈ halogéné, cycloalkyle en C₃₋₈, aryle, aralkyle ; et R¹⁰ et R¹¹ peuvent former ensemble un noyau à 5 à 8 chaînons ;
R² est un groupe alkyle en C₁₋₈ ou cycloalkyle en C₃₋₈, aryle ou aralkyle, ou encore deux radicaux R² forment ensemble dans la même molécule un noyau à 5 à 8 chaînons ;
R³ est un groupe alkyle en C₁₋₈, hétéroaryle, aryle, cycloalkyle en C₃₋₈, aralkyle et
Z¹ est un atome d'hydrogène ou un groupe alkyle en C₁₋₈, aralkyle, -CO₂R², -CON(R²)₂, -SO₂R¹⁰, -PO(R¹⁰)₂ ou -COR³.

2. Procédé selon la revendication 1, **caractérisé en ce que** l'hydrogénation asymétrique de composés de formule générale II est mise en oeuvre en présence d'une base servant d'additif.

3. Procédé de préparation d'un composé de formule I selon l'une des revendications 1 ou 2, **caractérisé en ce que** l'hydrogénation asymétrique a lieu dans une plage de températures de 10 à 200°C, sous une pression de 1 à 250 bar, avec un rapport du substrat au catalyseur de 20-80 000.

4. Procédé selon l'une des revendications 1 ou 2, **caractérisé en ce qu'**on utilise en tant qu'additifs des bases de l'ensemble comprenant les sels d'acides carboxyliques, les carbonates, les amines primaires, secondaires, tertiaires, et les imides.

5. Procédé selon l'une des revendications 2 ou 4, **caractérisé en ce qu'**on utilise en tant que bases des amines secondaires ou tertiaires.

6. Procédé selon l'une des revendications 1 ou 2, **caractérisé en ce que** la quantité de l'additif du groupe comprenant les sels d'acides carboxyliques, les amines primaires, secondaires et tertiaires, est comprise dans la plage de 0,001 à 100 équivalents en moles par rapport au composé selon la formule II.

7. Les composés
hydrogénosulfate de 1-(4-méthoxy-benzyl)-3,4,5,6,7,8-hexahydro-isoquinoléine,
hexafluorophosphate de 1-(4-méthoxy-benzyl)-3,4,5,6,7,8-hexahydro-isoquinoléine,
tétrafluoroborate de 1-(4-méthoxy-benzyl)-3,4,5,6,7,8-hexahydro-isoquinoléine,
phtalate de 1-(4-méthoxy-benzyl)-3,4,5,6,7,8-hexahydro-isoquinoléine,
perchlorate de 1- (4-méthoxy-benzyl)-3,4,5,6,7,8-hexahydro-isoquinoléine.
